# EUROPEAN PATENT APPLICATION

(11) **EP 4 319 170 A1**
(43) Date of publication of application: **07.02.2024**
(21) Application number: 22193088.6
(22) Date of filing: 31.08.2022
(51) Int. Cl.: H04N 21/431, H04N 21/436, H04N 21/4788, H04N 21/81, A61B 8/00, G16H 30/20, G16H 30/40, G16H 40/67, G16H 50/20, G16H 80/00

(54) **VENDOR-AGNOSTIC REMOTE-CONTROLLED SCREEN OVERLAY FOR COLLABORATION IN A VIRTUALIZED RADIOLOGY ENVIRONMENT**

(30) Priority: 04.08.2022 US 202263394988 P
(71) Applicant: Koninklijke Philips N.V., 5656 AG Eindhoven (NL)
(72) Inventor: AMTHOR, Thomas Erik, Eindhoven (NL)
(74) Representative: Philips Intellectual Property & Standards

(57) **Abstract**

A remote expert assists a local operator of a medical imaging device during a medical imaging examination. A user interface (UI) is provided on an assistor electronic device operable by the remote expert. The UI displays controller video output by an electronic controller of the medical imaging device. Graphical annotations for updating the controller video are received via at least one user input device of the assistor electronic device. The controller video overlaid with the graphical annotations is displayed on a controller display of the medical imaging device. In one approach, this entails splitting the controller video into a plurality of feeds, transmitting one of the feeds to the assistor electronic device, and transmitting another of the feeds to a screen overlay processor that also receives the graphical annotations from the assistor electronic device and that generates the controller video overlaid with the graphical annotations.

## Description

The following relates generally to the imaging arts, remote imaging assistance arts, remote imaging examination monitoring arts, and related arts.

### BACKGROUND OF THE INVENTION

Medical imaging, such as computed tomography (CT) imaging, magnetic resonance imaging (MRI), positron emission tomography (PET) imaging, fluoroscopy imaging, and so forth, is a critical component of providing medical care, and is used in a wide range of medical fields, such as cardiology, oncology, neurology, orthopedics, to name a few. The operator of the medical imaging device used to acquire the medical images is typically a trained technologist, while interpretation of the medical images is often handled by a medical specialist such as a radiologist. Interpretation of radiology reports or findings by the radiologist can be handled by the patient's general practitioner (GP) physician or a medical specialist such as a cardiologist, oncologist, orthopedic surgeon, or so forth.

Currently, medical imaging is in high demand. As the world population ages, the demand for quick, safe, high quality medical imaging will only continue to grow, putting further pressure on imaging centers and their staff. Under such conditions, errors can occur, and can often be costly. One approach for imaging centers to boost efficiency and grow operations at no extra labor costs is through a radiology operations command center (ROCC) system. Radiology operations command centers enable teams to work across the entire network of imaging sites, providing their expertise as needed and remotely assisting less experienced technologists in carrying out high quality scans. Remote technologists or experts can monitor the local operators of scanning procedures through cameras installed in the scanning areas (or from other sources, such as sensors (including radar sensors), console video feeds, microphones connected to Internet of Things (IoT) devices, and so forth. In addition, these sources can be supplemented by other data sources like Health-Level 7 (HL7), Digital Imaging and Communications in Medicine (DICOM), Electronic Health Record (EHR) databases, and so forth.

When collaborating via the ROCC platform, the local and remote technologists can communicate via an audio or video call on a tablet computer. In addition, the remote technologist can see the local console screen via video capture and transmission. Hence, the remote technologist can observe control or other actions taken by the local technologist, and can also observe the mouse pointer or other on-display user interfacing elements controlled by the local technologist.

In many situations, due to safety or security regulations, the remote technologist will only be allowed to view the local screen but not interact with the scanner system or local input device. Moreover, explanations of user interface (UI) interactions or identification of specific areas in an image must be conducted via audio communication, as the remote operator has no way of interacting with the local console screen. This makes assistive sessions with unexperienced local staff particularly difficult.

The following discloses certain improvements to overcome these problems and others.

### SUMMARY OF THE INVENTION

In one aspect, an assistive system is disclosed for providing remote assistance to a local operator of a medical imaging device that has an electronic imaging device controller and a controller display. The assistive system includes an assistor electronic device including an electronic processor, an assistor display, and at least one user input device. The assistive system further includes a video router configured to: receive controller video from the electronic imaging device controller; receive overlay content or video from the assistor electronic device; output the controller video to the assistor electronic device; and output annotated video to the controller display, whereby the annotated video is displayed on the controller display. The assistor electronic device is programmed to provide a graphical user interface (GUI) operative to display the controller video on the assistor display, and receive graphical annotations of the controller video from the at least one user input device. The assistor electronic device is further programmed to generate the overlay content or video comprising at least the graphical annotations. The annotated video comprises the controller video overlaid with the graphical annotations.

In another aspect, a method is disclosed of providing assistance from a remote expert to a local operator of a medical imaging device during a medical imaging examination. The method includes: providing a user interface (UI) on an assistor electronic device operable by the remote expert. The UI displays controller video output by an electronic controller of the medical imaging device. The method further includes receiving, via at least one user input device of the assistor electronic device, one or more graphical annotations to update the controller video. The controller video overlaid with the graphical annotations is displayed on a controller display of the medical imaging device.

In another aspect, an assistive system is disclosed for providing remote assistance to a local operator of a medical imaging device having an electronic imaging device controller and a controller display. The assistive system includes an assistor electronic device and a video router. The assistor electronic device includes an electronic processor, an assistor display, and at least one user input device. The video router is configured to: receive controller video from the electronic imaging device controller; receive graphical annotations from the assistor electronic device; output the controller video to the assistor electronic device; and output annotated video to the controller display, whereby the annotated video is displayed on the controller display. The assistor electronic device is programmed to: provide a GUI operative to display the controller video on the assistor display; and receive the graphical annotations of the controller video from the at least one user input device. The annotated video comprises the controller video overlaid with the graphical annotations.

One advantage resides in providing a remote expert or radiologist assisting a technologist in conducting a medical imaging examination with a capability to annotate a screen operable by the technologist.

Another advantage resides in remote expert or radiologist assisting a technologist in conducting a medical imaging examination with a capability to annotate a screen operable by the technologist by without interfering with the local imaging system or host computer.

Another advantage resides in creating screen overlays generated by the remote expert or radiologist directly on the video signal of the local console monitor.

Another advantage resides in creating such screen overlays controlled by a remote operator in real time.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIG. 1 diagrammatically shows an illustrative apparatus for providing remote assistance in accordance with the present disclosure.
FIG. 2 shows another aspect of the apparatus of FIG. 1.
FIG. 3 shows an example flow chart of operations suitably performed by the apparatus of FIG. 1.
FIG. 4 diagrammatically shows some examples of highlighting overlay elements.
FIGS. 5, 6, and 7 show alternative embodiments to the embodiment shown in FIG. 2.

### DETAILED DESCRIPTION OF EMBODIMENTS

In some current implementations of a Remote Operations Control Center (ROCC), the remote expert receives a scraped copy of the controller screen, and thus can see what a local operator sees. The scraped controller screen viewed by the remote expert typically includes a mouse pointer or other user interfacing element(s) controlled by the local operator, so the local operator may be able to point out features on the screen during communications with the remote expert. However, there is no mechanism by which the remote expert can point to features on the controller screen in a way that the local operator can see.

Embodiments disclosed herein remedy this deficiency by providing a mechanism for the remote expert to draw annotations on the controller display that are visible to the local operator as an overlay on the controller display. To enable this to be vendor-agnostic, in some embodiments the approach operates on the video signal, intercepting it as it passes from the imaging device controller to the display viewed by the local operator and modifying the video signal to add the overlay.

Some existing ROCC implementations employ a Keyboard, Video, & Mouse (KVM) switch to act as a transmitter of the video signal, and a video splitter is configured to split the video signal to two paths: the path to the video display seen by the local operator, and a second path providing the scraped screen that is sent to the remote expert. To implement the overlay disclosed herein, an additional screen overlay processor is interposed in the path from the KVM switch (or more generally, video router) to the local operator display. The operation of the screen overlay processor depends on the nature of the video signal. For a digital video signal such as HMDI, DisplayPort, or digital DVI, the screen overlay processor can be implemented as a digital signal processing (DSP) component, e.g. using a graphical processing unit (GPU) and hardware acceleration to provide sufficient speed in some embodiments. On the other hand, if the video signal is analog, such as an analog DVI signal, then an additional analog-to-digital (A/D) frontend and digital-to-analog (D/A) backend is suitably employed, with DSP being performed on the digitized display content output by the A/D frontend prior to the subsequent D/A backend. In some embodiments, the annotated overlay elements may be received from the remote expert as vectorized or symbolic graphical elements, e.g. represented as lines or shapes delineated by coordinates, along with color and transparency values. The DSP then converts these to a screen overlay that is fused with the received controller display video.

At the remote expert workstation, suitable hardware and software is added to implement the screen annotation user interface (UI). For example, the workstation can have a touch-sensitive display enabling the remote expert to draw on the window presenting the controller display, and/or a mouse pointer, touchpad, or the like can be used for this purpose. As noted, the remote expert workstation in some embodiments converts these annotations to the vectorized or symbolic graphical elements that are sent via the Internet to the screen overlay processor.

One potential concern is that the annotations added by the remote expert could confuse or distract the local operator. To address this potential concern, in some embodiments a "kill switch" is provided at the local operator side that can enable the local operator to turn off the annotations overlay in the interest of safety. As another contemplated variant, additional annotations could be overlaid on the local operator screen beyond the annotations directed created by the remote expert, such as a red box around the entire controller display indicating the remote operator is viewing it.

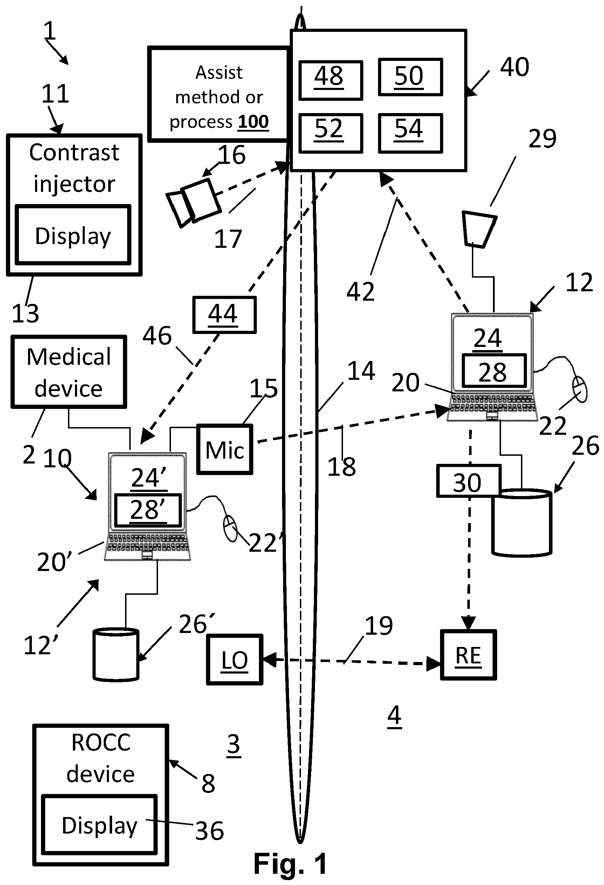

With reference to FIG. 1, an apparatus **1** for providing assistance from a remote medical imaging expert **RE** (or supertech) to a local technologist operator **LO** is shown. As shown in FIG. 1, the local operator **LO,** who operates a medical imaging device (also referred to as an image acquisition device, imaging device, and so forth) 2, is located in a medical imaging device bay 3, and the remote expert **RE** is disposed in a remote service location or center **4.** It should be noted that the "remote expert" **RE** may not necessarily directly operate the medical imaging device **2,** but rather provides assistance to the local operator **LO** in the form of advice, guidance, instructions, or the like. The remote location **4** can be a remote service center, a radiologist's office, a radiology department, and so forth. The remote location **4** may be in the same building as the medical imaging device bay **3** (this may , for example, in the case of a "remote operator or expert" **RE** who is a radiologist tasked with peri-examination image review), but more typically the remote service center **4** and the medical imaging device bay **3** are in different buildings, and indeed may be located in different cities, different countries, and/or different continents. In general, the remote location **4** is remote from the imaging device bay **3** in the sense that the remote expert **RE** cannot directly visually observe the imaging device **2** in the imaging device bay **3** (hence optionally providing a video feed as described further herein).

The image acquisition device **2** can be a Magnetic Resonance (MR) image acquisition device, a Computed Tomography (CT) image acquisition device; a positron emission tomography (PET) image acquisition device; a single photon emission computed tomography (SPECT) image acquisition device; an X-ray image acquisition device; an ultrasound (US) image acquisition device; or a medical imaging device of another modality. The imaging device **2** may also be a hybrid imaging device such as a PET/CT or SPECT/CT imaging system. While a single image acquisition device **2** is shown by way of illustration in FIG. 1, more typically a medical imaging laboratory will have multiple image acquisition devices, which may be of the same and/or different imaging modalities. Moreover, the remote service center **4** may provide service to multiple hospitals. The local operator controls the medical imaging device **2** via an electronic imaging device controller **10.** The remote operator is stationed at an assistance electronic device **12** (or, more generally, a remote workstation **12** or an electronic controller **12).**

To provide for contrast-enhanced imaging in certain imaging modalities, an optional contrast injector **11** is configured to inject the patient with a contrast agent. The contrast injector **11** is a configurable automated contrast injector having a display **13.** The user (usually the imaging technologist) loads a vial or syringe of contrast agent (or two, or more, vials of different contrast agent components) into the contrast injector **11,** and configures the contrast injector **11** by entering contrast injector settings such as flow rates, volumes, time delays, injection time durations, and/or so forth via a user interface (UI) of the contrast injector **11.** The UI may be a touch-sensitive overlay of the display **13,** and/or physical buttons, keypad, and/or so forth. In a variant embodiment, the optional contrast injector **11** is integrated with the imaging device controller **10** (e.g., via a wired or wireless data connection), and the contrast injector **11** is controlled via the imaging device controller **10,** including displaying the contrast injector settings in a (optionally selectable) window on the display of the imaging device controller **10.**

As diagrammatically shown in FIG. 1, in some embodiments, a camera **16** (e.g., a video camera) is arranged to acquire a video stream or feed **17** of a portion of a workspace of the medical imaging device bay **3** that includes at least the area of the imaging device **2** where the local operator **LO** interacts with the patient, and optionally may further include the imaging device controller **10.** In some embodiments, a microphone **15** is arranged to acquire an audio stream or feed **18** of the workspace so that the remote expert has auditory situational awareness as well. The video stream 17 and/or the audio stream 18 is sent to the assistance electronic device **12** via the communication link **14,** e.g. as a streaming video feed received via a secure Internet link.

The communication link **14** also provides a natural language communication pathway **19** for verbal and/or textual communication between the local operator and the remote operator. For example, the natural language communication link **19** may be a Voice-Over-Internet-Protocol (VOIP) telephonic connection, an online video chat link, a computerized instant messaging service, or so forth. Alternatively, the natural language communication pathway **19** may be provided by a dedicated communication link that is separate from the communication link **14** providing the data communications **17, 18,** e.g. the natural language communication pathway **19** may be provided via a landline telephone. In some embodiments, the natural language communication link **19** allows a local operator **LO** to call a selected remote expert **RE.** The call, as used herein, can refer to an audio call (e.g., a telephone call), a video call (e.g., a Skype or Facetime or other screen-sharing program), or an audio-video call. In another example, the natural language communication pathway **19** may be provided via a local electronic processing device, for example comprising an ROCC device **8,** such as a mobile device (e.g., a tablet computer or a smartphone), or can be a wearable device worn by the local operator **LO,** such as an augmented reality (AR) display device (e.g., AR goggles), a projector device, a heads-up display (HUD) device, etc., each of which having a display device **36.** For example, an "app" can run on the ROCC device **8** (operable by the local operator **LO)** and the assistance electronic device **12** (operable by the remote expert **RE)** to allow communication (e.g., audio chats, video chats, and so forth) between the local operator and the remote expert.

FIG. 1 also shows, in the remote service center **4** including the assistance electronic device **12,** such as an electronic processing device, a workstation computer, or more generally a computer, which is operatively connected to receive and present the video feed **17** of the medical imaging device bay **3** from the camera **16** and/or to the audio feed **18.** Additionally or alternatively, the assistance electronic device **12** can be embodied as a server computer or a plurality of server computers, e.g. interconnected to form a server cluster, cloud computing resource, or so forth. The workstation **12** includes typical components, such as an electronic processor **20** (e.g., a microprocessor), at least one user input device (e.g., a mouse, a keyboard, a trackball, and/or the like) **22,** and at least one display device **24** (e.g. an LCD display, plasma display, and/or so forth). In some embodiments, the display device **24** can be a separate component from the workstation **12.** The display device **24** may also comprise two or more display devices. The electronic processor **20** is operatively connected with a one or more non-transitory storage media **26.** The non-transitory storage media **26** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the workstation **12,** various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a graphical user interface (GUI) **28** for display on the remote operator display device **24.** The video feed **17** from the camera **16** can also be displayed on the display device **24,** and the audio feed **18** can be output on the assistance electronic device **12** via a loudspeaker **29.** In some examples, the audio feed **18** can be an audio component of an audio/video feed (such as, for example, recording as a video cassette recorder (VCR) device would operate).

The medical imaging device controller **10** in the medical imaging device bay **3** also includes similar components as the assistance electronic device **12** disposed in the remote service center **4.** Except as otherwise indicated herein, features of the medical imaging device controller **10,** which includes a local workstation **12',** disposed in the medical imaging device bay **3** similar to those of the assistance electronic device **12** disposed in the remote service center **4** have a common reference number followed by a "prime" symbol, and the description of the components of the medical imaging device controller **10** will not be repeated. In particular, the medical imaging device controller **10** is configured to display a GUI **28'** on a display device or controller display **24'** that presents information pertaining to the control of the medical imaging device **2,** such as configuration displays for adjusting configuration settings an alert **30** perceptible at the remote location when the status information on the medical imaging examination satisfies an alert criterion of the imaging device **2,** imaging acquisition monitoring information, presentation of acquired medical images, and so forth. It will be appreciated that the screen mirroring data stream **18** carries the content presented on the display device **24'** of the medical imaging device controller **10.** The communication link **14** allows for screen sharing between the display device **24** in the remote service center **4** and the display device **24'** in the medical imaging device bay **3.** The GUI **28'** includes one or more dialog screens, including, for example, an examination/scan selection dialog screen, a scan settings dialog screen, an acquisition monitoring dialog screen, among others. The GUI **28'** can be included in the video feed **17** and displayed on the assistance electronic device display **24** at the remote location **4.**

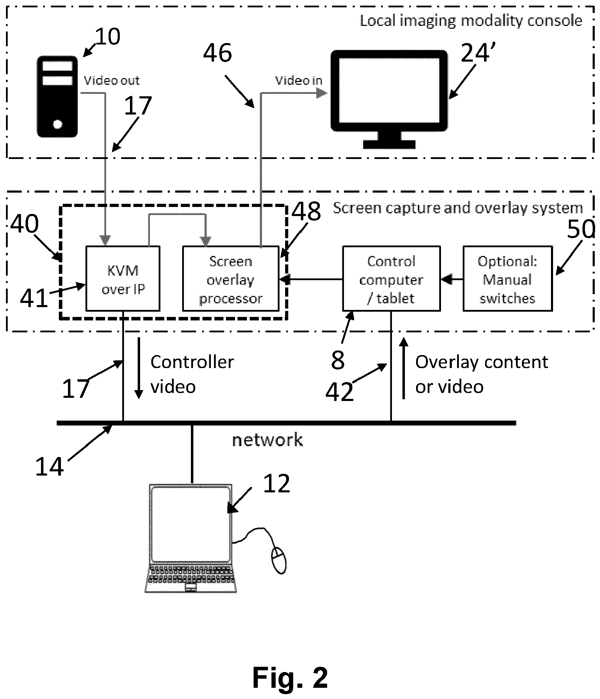

With reference to FIG. 2, and with continuing reference to FIG. 1, the apparatus 1 includes a video router **40** (diagrammatically shown as a rectangle in FIG. 1). In some examples, the video router **40** comprises a keyboard, video, monitor (KVM) switch **41.** In other examples, the video router **40** may be constructed of a set of discrete video signal splitter and combiner components. The video router **40** is configured to receive a controller video **17** from the electronic imaging device controller **10,** and also receive an overlay content or video **42** from the assistor electronic device **12.** The controller video **17** is output to the assistor electronic device **12,** where it is displayed on the display device **24** via the GUI **28** to allow the assistor to annotate the controller video displayed on the assistor electronic device **12.**

The assistor electronic device **12** used by the remote expert **RE** is then programmed to provide inputs via the at least one user input device **22** indicative of graphical annotations **44** of the controller video **17.** The graphical annotations **44** can include, for example, one or more of vectorized or symbolic graphical elements, bitmapped graphical elements, color elements, or transparency values. From this, the assistor electronic device **12** is configured to generate overlay content or video **42** that is, or is used to generate, annotated video **46** comprising at least the graphical annotations **44** to the controller display **24'** of the electronic imaging device controller **10.** The annotated video **46** thus comprises the controller video **17** overlaid with the graphical annotations **44.**

In one embodiment, the overlay content or video **42** is generated at the assistor electronic device **12** as the annotated video **46** comprising the controller video **17** overlaid with the graphical annotations **44,** and the video router **40** is configured to output the annotated video **46** received from the assistor electronic device **12** to the controller display **24'.** That is, the operation of combining the controller video **17** and the graphical annotations **44** is in this embodiment performed at the assistor electronic device **12** located with the remote expert **RE.**

In another embodiment, the overlay content or video **42** is generated as the graphical annotations **44** without the controller video **17,** and the video router **40** includes a digital signal processor (DSP) **48** (identified as a screen overlay processor in FIG. 2) programmed to generate the annotated video **46** by overlaying the graphical annotations **44** received from the assistor electronic device **12** onto the controller video **17.** That is, the operation of combining the controller video **17** and the graphical annotations **44** is in this embodiment performed at the video router **40** which is located with the local operator **LO.** In this embodiment, the overlay content or video **42** received at the video router **40** represents the graphical annotations **44.** In one implementation, the overlay content or video **42** transmitted from the assistor electronic device **12** to the video router **40** is overlay content comprising the graphical annotations **44** represented as vectorized or symbolic graphical elements, e.g. represented as lines or shapes delineated by coordinates, along with color and transparency values. In this implementation, the DSP **48** of the video router **40** then converts these received vectorized or symbolic graphical elements to a screen overlay that is then fused with the controller video **17** to produce the annotated video **46.** In another implementation, the overlay content or video **42** transmitted from the assistor electronic device **12** to the video router **40** comprises overlay video constructed as the graphical annotations **44** converted to bitmapped video containing only the graphical annotations. In this case the DSP **48** of the video router **40** then fuses the overlay video (which here contains only the annotations **44)** and the controller video **17** to produce the annotated video **46.** In either of these two implementations, the kill switch **50** can be implemented at the video router **40** by ceasing to fuse the received overlay content or video **42** with the controller video 17.

In the illustrative example of FIG. 2, the video router **40** is configured to receive the overlay content or video **42** via the assistor electronic device **12** via the ROCC device **8.** However, in other embodiments the video router **40** may receive the overlay content or video **42** directly from the network **14,** without the ROCC device **8** serving as a relay.

In some embodiments, the video router **40** also includes a manual overlay disable switch **50** operable by the local operator **LO** to switch the video router **40** from outputting the annotated video **46** to the controller display **24'** to outputting the controller video **17** to the controller display **24'.** In variant embodiments in which the controller video **17** and the graphical annotations **44** are combined at the assistor electronic device **12** located with the remote expert **RE,** implementation of the kill switch **50** would entail sending the kill signal to the assistor electronic device **12,** and the assistor electronic device **12** would then not add the annotations **44** into the controller video **17** in response to activation of the kill switch **50.**

Furthermore, as disclosed herein, the ROCC device **8,** the assistor electronic device **12,** and the video router **40** are configured to perform a method or process **100** for providing assistance during a medical imaging examination performed using a medical imaging device **2** (i.e., by assisting local operators **LO** of respective medical imaging devices **2** during medical imaging examinations by a remote expert **RE).** The instructions to perform the method **100** are stored in the non-transitory computer readable medium **26** of the assistance electronic device **12,** in the video router **40,** and in the ROCC device **8.**

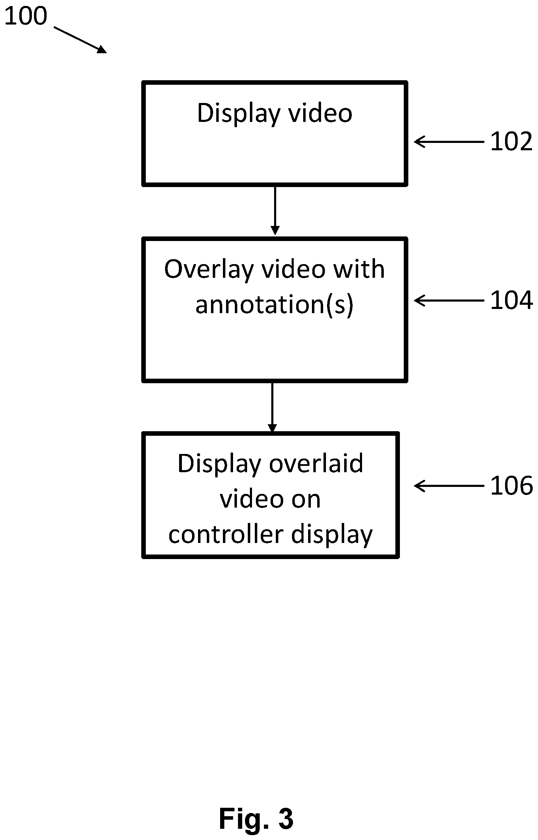

With reference to FIG. 3, and with continuing reference to FIGS. 1 and 2, an illustrative embodiment of the method **100** is diagrammatically shown as a flowchart. To begin the method **100,** an imaging examination is commenced by the local operator **LO** using the medical imaging device **2.** An event can occur during the examination which requires assistance from a remote expert **RE.** The controller video feed (acquired by the one or more cameras **16)** is routed to the video router **40.**

At an operation **102,** the GUI **28** is provided on the assistor electronic device **12,** and the controller video **17** is displayed thereon. At an operation **104,** one or more graphical annotations **44** are received by the assistor electronic device **12** (i.e., input by the remote expert **RE** via the at least one user input device **22)** and overlaid onto the controller video **17** to generate the annotated video **46.** In some embodiments, the controller video **17** overlaid with the graphical annotations **44** is generated by a screen overlay component **48** that is part of the video router **40** and comprises a digital signal processing (DSP) component.

At an operation **106,** the annotated video **46** is displayed on the controller display **24'** of the medical imaging device controller **10.** In some embodiments, the controller video **17** is split i.e., via the KVM switch **41,** or using a discrete video splitter) to split the controller video **17** into a plurality of feeds. One of the feeds is transmitted to the assistor electronic device **12,** Another one of the feeds is transmitted to the screen overlay processor **48** which also receives the graphical annotations 44 from the assistor electronic device **12,** that generates the controller video **17** overlaid with the graphical annotations **44** to generate the annotated video **46.**

In another embodiment, the controller video **17** is an analog video, and the controller video **17** is converted to digital controller video that is processed by the DSP component **48** using an analog-to-digital converter (ADC) **52** implemented in the video router **40.** The controller video **17** overlaid with the graphical annotations **44** (i.e., the annotated video **46)** is converted to an analog signal via a digital-to-analog converter (DAC) **54** implemented in the video router **40.**

In another embodiment, the displaying of the controller video **17** overlaid with the graphical annotations **44** on the controller display **24'** is switched to displaying the controller video **17** without the graphical annotations **44** on the controller display **24** in response to activation of the overlay disable switch **50** by the local operator **LO.**

In another embodiment, the annotated video **46** is displayed on the display device **36** of the ROCC device **8,** and the graphical annotation(s) **44** are received from the local operator **LO,** in which the annotated video **46** includes the graphical annotation(s) **44** received from the local operator **LO.**

The system may offer the local operator **LO** a kill switch **50** to switch screen overlays on and off. This kill switch **50** can be realized by a touch or click button or switch displayed on a separate screen, or by a physical manual button or switch **50.** The kill switch **50** enables the local operator **LO** to avoid distraction by stopping the process of overlaying the annotations **44** on the controller video **17.**

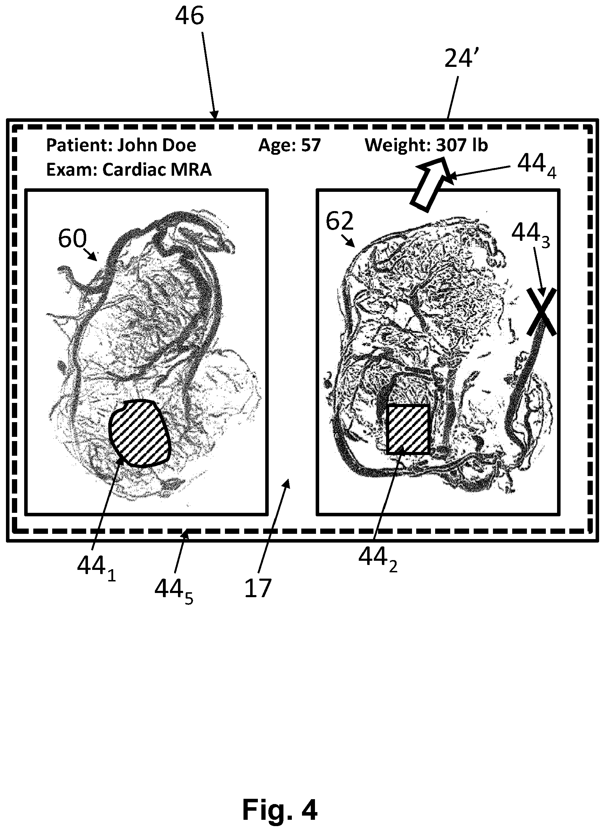

With reference to FIG. 4, some examples of highlighting overlay elements drawn by the remote expert **RE** are diagrammatically shown. FIG. 4 shows magnetic resonance angiography (MRA) images **60** and **62** such as might be displayed on the controller display **24'** during an MRA imaging session. It is to be appreciated that this is merely a nonlimiting illustrative example, and the disclosed approaches for remote expert annotation can be more generally employed in any medical imaging context (MR, CT, PET, et cetera). The display can also include text fields, such as the nonlimiting illustrative examples of "Patient: John Doe," "Age: 57", "Weight: 307 lb.", and "Exam: Cardiac MRA". FIG. 4 shows an example of the annotated video **46** including the combination of the controller video **17** and the graphical annotations **44₁, 44₂, 44₃, 44₄,** and **44s** drawn by the remote expert **RE** using the assistor electronic device **12.** The overlay elements **44₁** and **44₂** are areas highlighted by the remote expert **RE,** while the annotation **44₃** is a location marker shown in FIG. 4 as an "X", although other types of location markers are contemplated. The area overlay elements **44₁** and **44₂** can, for example, be hand drawn by the remote expert **RE** using a mouse pointer, touch-sensitive display, or the like. The annotation **44₄** is a superimposed graphical annotation cursor **44₄** operable by the remote expert **RE** to point out objects. In the illustrative example, the remote expert **RE** is using the superimposed graphical annotation cursor **44₄** to highlight the text "Weight: 307 lb.", possibly as part of explaining that the imaging subject's weight may explain some aspect of the imaging examination. As can be seen, the various graphical annotations **44₁, 44₂, 44₃,** and **44₄** drawn by the remote expert **RE** enable the remote expert to identify features of images, text, or other content shown on the controller display **24',** thus assisting in conveying information to the local operator **LO.** Overlay elements are preferably displayed in a transparent way so that information on the ROCC device **8** below the overlay elements is still visible to the local operator **LO.** Some examples of functional overlays shown in FIG. 4 include a border annotation **44s** to indicates that this ROCC device **8** is being watched by the remote expert **RE,** and the pointer symbol **44₄** already described that mimics the mouse movement of the remote expert **RE** on the shared screen. The border annotation **44s** is diagrammatically shown in FIG. 4 as a dashed line, but could be implemented for example as a red border to indicate the RE is observing.

The remote expert **RE,** viewing the medical imaging device controller **10** via the KVM **41,** will need to interact with the video feed **17** to define screen overlay elements. For this reason, the assistance electronic device **12** includes a drawing tool for screen overlay elements. The drawing tool allows the remote expert **RE** to draw overlay elements directly onto the image of the shared screen.

The software then transforms the screen coordinates of the elements to the screen coordinates of the medical imaging device controller **10** (in case the screen resolutions or aspect ratios differ). Alternatively, the software calculates relative coordinates with respect to the screen extensions and leaves the determination of absolute coordinates to the ROCC device **8** or the screen overlay processor **48.**

All information required for generating the overlays is transmitted through the network connection **14** to the ROCC device **8** in real time. This information includes, but is not limited to, coordinates, shape, color, and transparency level of all overlay elements; coordinates, shape, color, and transparency level of pointer, status information for additional functional overlays, such as activity of screen sharing, and so forth.

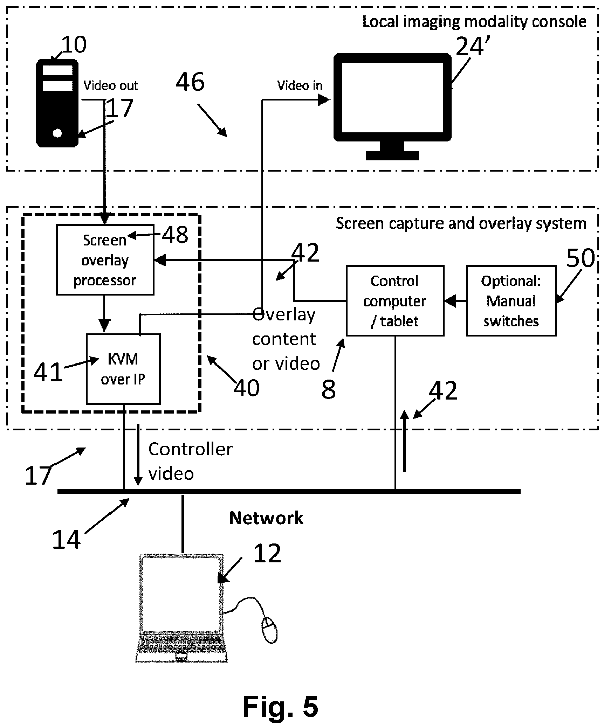

With reference to FIGS. 5-7, some alternative embodiments to the embodiment shown in FIG. 2 are presented. Each of these embodiments includes components analogous to those of FIG. 2 which are indicated by like reference numbers. As with the embodiment of FIG. 2, each of the embodiments of FIGS. 5-7 includes a video router **40** that receives controller video **17** from the electronic imaging device controller **10** and overlay content or video **42** from the assistor electronic device **12,** and the video router **40** outputs the controller video **17** to the assistor electronic device **12,** and also outputs the annotated video **46** to the controller display **24',** whereby the annotated video **46** is displayed on the controller display **24'.**

In the embodiment of FIG. 5, the overlay content or video **42** is constructed before transmission via the KVM **41.** This has the advantage that the remote operator using the assistor electronic device **12** sees exactly what the local operator sees on the controller display **24'.** One possible disadvantage of the approach of FIG. 5 is that drawing elements may seem to have to some delay due to network latency.

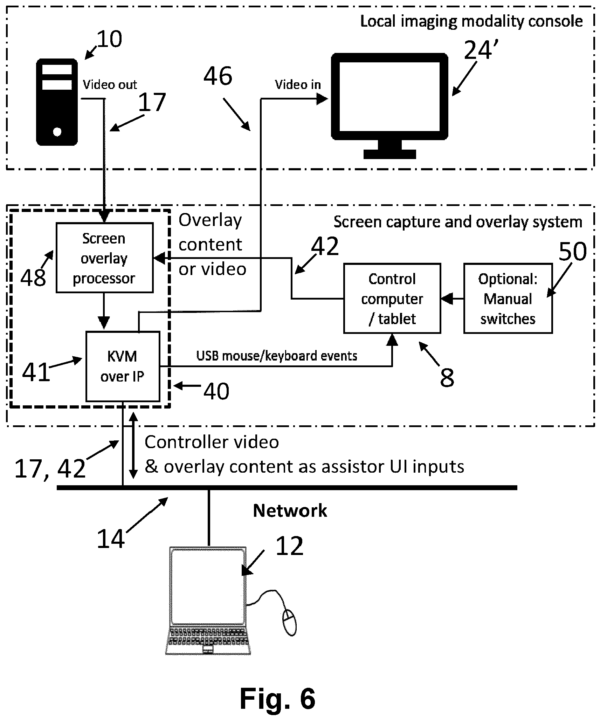

The embodiment of FIG. 6 is similar to that of FIG. 5, but in the embodiment of FIG. 6 the overlay content or video **42** is in the form of overlay content **42** represented by UI inputs at the assistor electronic device **12** (e.g., keyboard and mouse inputs **42)** made by the operator of the assistor electronic device **12** in drawing, typing, or otherwise creating the graphical annotations **44.** These UI inputs forming the overlay content **42** are transmitted through the KVM **41** and fed to the ROCC device **8** which generates the overlay content or video **42** that is then input to the video router **40.**

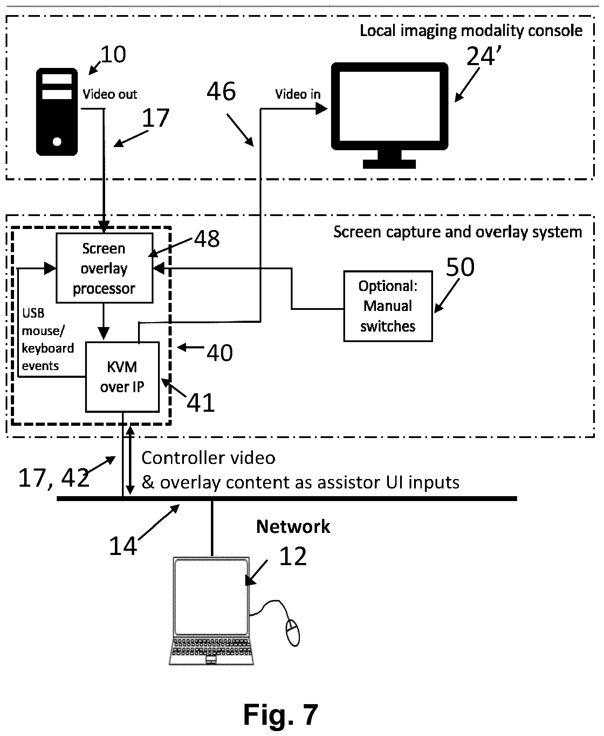

In the embodiment of FIG. 7, a similar system to that of FIG. 6 is shown, except that in FIG. 7 the UI inputs forming the overlay content **42** are input directly to the screen overlay processor **48** (rather than to the ROCC device **8),** and the screen overlay processor **48** interprets the UI inputs forming the overlay content **42** directly to produce the annotated video **46** which is then fed to the controller display **24'** via the KVM **41.**

In the following, some further variant embodiments are described.

In some embodiments, the graphical overlay elements have additional parameters controlling animations, flashing, etc. to increase visibility.

In some embodiments, the remote expert **RE** or the ROCC device **8** must request the sharing of overlays. The local operator **LO** must then acknowledge the request before the screen overlay functionality is activated.

In some embodiments, the local operator **LO** can interact with the overlay elements on the ROCC device **8** using the local pointer device (mouse etc.). To enable this, the movements of the local pointing device must be processed by the ROCC device **8** or the screen overlay processor **48.** The KVM **41,** being connected not only to the annotation(s) **44,** but also to the pointing device, can facilitate this functionality.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. An assistive system **(1)** for providing remote assistance to a local operator **(LO)** of a medical imaging device **(2)** having an electronic imaging device controller **(10)** and a controller display **(24'),** the assistive system comprising:
an assistor electronic device **(12)** including an electronic processor **(20),** an assistor display **(24),** and at least one user input device **(22);**
a video router **(40)** configured to:
receive controller video **(17)** from the electronic imaging device controller;
receive overlay content or video **(42)** from the assistor electronic device;
output the controller video to the assistor electronic device; and
output annotated video **(46)** to the controller display, whereby the annotated video is displayed on the controller display;
wherein the assistor electronic device is programmed to:
provide a graphical user interface (GUI) **(28)** operative to display the controller video on the assistor display; and
receive graphical annotations **(44)** of the controller video from the at least one user input device, the assistor electronic device further programmed to generate the overlay content or video **(42)** comprising at least the graphical annotations; and
wherein the annotated video comprises the controller video overlaid with the graphical annotations.

2. The assistive system **(1)** of claim 1, wherein the assistor electronic device **(12)** is programmed to generate the overlay content or video **(42)** as the annotated video **(46)** comprising the controller video **(17)** overlaid with the graphical annotations **(44),** and the video router **(40)** is configured to output the annotated video received from the assistor electronic device to the controller display **(24').**

3. The assistive system **(1)** of claim 1, wherein the assistor electronic device **(12)** is programmed to generate the overlay content or video **(42)** comprising the graphical annotations **(44)** without the controller video **(17),** and the video router **(40)** includes a digital signal processor **(48)** programmed to generate the annotated video **(46)** by combining the overlay content or video **(42)** received from the assistor electronic device with the controller video **(17).**

4. The assistive system **(1)** of claim 3, wherein the overlay content or video **(42)** received from the assistor electronic device **(12)** is overlay content in which the graphical annotations **(44)** are represented as vectorized or symbolic graphical elements, and the digital signal processor **(48)** is programmed to generate the annotated video **(46)** by converting the vectorized or symbolic graphical elements to a screen overlay and fusing the screen overlay with the controller video **17** to produce the annotated video **(46).**

5. The assistive system **(1)** of claim 3, wherein the overlay content or video **(42)** received from the assistor electronic device **(12)** is overlay video in which the graphical annotations **(44)** are represented as overlay video, and the digital signal processor **(48)** is programmed to generate the annotated video **(46)** by fusing the overlay video with the controller video **17** to produce the annotated video **(46).**

6. The assistive system **(1)** of any one of claims 4-5, wherein the video router **(40)** includes an overlay disable switch **(50)** operable by the local operator **(LO)** to cause the video router **(40)** to stop combining the overlay content or video **(42)** received from the assistor electronic device with the controller video **(17).**

7. The assistive system **(1)** of any one of claims 1-6, further comprising:
a local electronic device **(8)** operable by the local operator **(LO),** wherein the video router **(40)** is configured to receive the overlay content or video **(42)** from the assistor electronic device **(12)** via the local electronic device and to output the controller video **(17)** to the assistor electronic device via the local electronic device.

8. The assistive system **(1)** of any one of claims 1-7, wherein the video router **(40)** comprises a keyboard, video, monitor (KVM) switch **41.**

9. A method **(100)** of providing assistance from a remote expert **(RE)** to a local operator **(LO)** of a medical imaging device **(2)** during a medical imaging examination, the method comprising:
providing a user interface (UI) **(28)** on an assistor electronic device **(12)** operable by the remote expert, the UI displaying controller video **(17)** output by an electronic controller **(10)** of the medical imaging device;
receiving, via at least one user input device **(22)** of the assistor electronic device, one or more graphical annotations **(44)** to update the controller video; and
displaying the controller video overlaid with the graphical annotations on a controller display **(24')** of the medical imaging device.

10. The method **(100)** of claim 9, further including:
splitting the controller video **(17)** into a plurality of feeds;
transmitting one of the feeds to the assistor electronic device **(12);** and
transmitting another of the feeds to a screen overlay processor **(48)** that also receives the graphical annotations **(44)** from the assistor electronic device and that generates the controller video (17) overlaid with the graphical annotations.

11. The method **(100)** of claim 10, wherein the splitting comprises employing a Keyboard, Video, & Mouse (KVM) switch **(41)** to split the feed **(17)** into the plurality of feeds.

12. The method **(100)** of any one of claims 9-11, wherein the assistor electronic device **(12)** implements a screen overlay component **(48)** configured to generate the controller video **(17)** overlaid with the graphical annotations **(44).**

13. The method **(100)** of any one of claims 9-12, wherein the controller video **(17)** overlaid with the graphical annotations **(44)** is generated by a screen overlay component **(48)** that comprises a digital signal processing (DSP) component.

14. The method **(100)** of claim 13, wherein the controller video **(17)** is an analog signal, the method further comprising:
converting the controller video to digital controller video that is processed by the DSP component **(48)** using an analog-to-digital converter (ADC) **(52);** and
converting the controller video overlaid with the graphical annotations **(44)** to an analog signal that is displayed by the controller display **(24')** using a digital-to-analog converter (DAC) **(54).**

15. The method **(100)** of any one of claims 9-14, wherein the graphical annotations **(44)** comprise one or more of vectorized or symbolic graphical elements, color elements, or transparency values.
